# EUROPÄISCHE PATENTSCHRIFT

(11) **EP 3 064 123 B1**
(45) Veröffentlichungstag und Bekanntmachung des Hinweises auf die Patenterteilung: **18.03.2020**
(21) Anmeldenummer: 16158046.9
(22) Anmeldetag: 01.06.2010
(51) Int. Cl.: A61B 1/07, G02B 6/00, G02B 23/24, A61B 1/00

(54) **ENDOSKOPROHR**
ENDOSCOPE TUBE
TUBE D'ENDOSCOPE

(30) Priorität: 17.06.2009 DE 102009025659
(43) Veröffentlichungstag der Anmeldung: 07.09.2016
(62) Teilanmeldung aus: 10005691.0
(73) Patentinhaber: Karl Storz SE & Co. KG, 78532 Tuttlingen (DE)
(72) Erfinder: Lei, Fang, 78048 VS-Villingen (DE); Weiger, Ulrich, 78600 Kolbingen (DE)

(56) Entgegenhaltungen:
- WO-A1-99/39135
- DE-A1- 2 232 582
- DE-A1- 2 328 554
- DE-A1- 3 718 609
- US-A- 4 697 577
- US-A- 5 617 498
- US-A- 5 947 958

## Beschreibung

Die Erfindung betrifft ein Endoskoprohr. Endoskoprohre sind Teile von Endoskopen, die insbesondere im Bereich der minimalinvasiven Chirurgie von Menschen oder auch von Tieren und ausnahmsweise auch im Bereich der Technik Anwendung finden.

Es sind Endoskoprohre bekannt, die rohrartig von ausgeprägter Länge ausgebildet sind und die an ihrem distalen Ende mit einem Beobachtungsfenster versehen sind, durch das im Operationsfall das vor dem distalen Ende befindlichen Operationsbereich eingesehen werden kann. Durch fakultativ zusätzlich vorgesehene Arbeitskanäle im Endoskoprohr werden endoskopische Instrumente eingeführt, mittels derer die chirurgische Manipulation im Operationsbereich vorgenommen wird. Zusätzlich weist das Ende des Endoskoprohres eine oder mehrere Lichtaustrittsöffnungen auf, die um das runde Beobachtungsfenster und die Öffnungen der Arbeitskanäle herum angeordnet sind. Es ist bekannt, die Lichtaustrittsöffnungen zur Aufnahme von Lichtleitern vorzusehen, aus deren Endflächen Licht austritt, so dass eine verlässliche Beleuchtung des Arbeitsbereiches gegeben ist. Das Beobachtungsfenster muss nicht notwendiger Weise kreisrund ausgebildet sein. Diese bekannten Endoskoprohre zeigen gerade bei der Verwendung von nicht kreisrunden Beobachtungsfenstern typischerweise ein unangenehm inhomogene Ausleuchtung des Arbeitsbereiches auf.

Der Erfindung liegt daher die Aufgabe zugrunde, ein Endoskoprohr anzugeben, das durch geeignete Maßnahmen sicherstellt, dass die Homogenität der Ausleuchtung verbessert wird, wobei dies unter Berücksichtigung des Querschnitts des Endoskoprohres und der Gestaltung des distalen Endes des Endoskoprohres im Hinblick auf eine verlässliche und sichere Handhabung im chirurgischen Einsatz haben soll.

Diese Aufgabe wird gelöst durch ein Endoskoprohr mit den Merkmalen des Patentanspruches 1. Vorteilhafte Weiterbildungen des erfindungsgemäßen Endoskoprohrs sind Gegenstand der Unteransprüche.

Als überraschend hat sich herausgestellt, dass durch geeignete Wahl der Anordnung bzw. Ausbildung der Lichtaustrittsöffnungen um das zentral am distalen Ende des Endoskoprohrs angeordnete langgestreckte Beobachtungsfenster eine deutlich verbesserte und weitgehend homogene Ausleuchtung des beobachteten Raumwinkelbereichs, der nicht notwendigerweise den vollen optischen Erfassungsbereich enthalten muss, ermöglicht.

Dabei hat es sich als besonders wirksam herausgestellt, die vorhandenen Lichtaustrittsöffnungen entlang der Längsausdehnung des Beobachtungsfensters verteilt anzuordnen, wobei die Verteilung unsymmetrisch bezüglich der Längsachse des Beobachtungsfensters erfolgen soll. Durch diese unsymmetrische, d.h. ungleichmäßige Anordnung der Lichtaustrittsöffnungen gelingt es eine sehr verteilte homogenisierte Verteilung zu ermöglichen. Dies insbesondere dann, wenn entlang der Länge des Beobachtungsfensters eine abwechselnde Anordnung der Lichtaustrittsfenster einmal rechts gefolgt von einmal links und wieder gefolgt von rechts, usw. gewählt ist und dabei insbesondere zusätzlich die Abstände zur Längsmittelachse des Beobachtungsfensters bzw. die Abstände entlang der Länge variiert werden bzw. die Fläche der Lichtaustrittsöffnungen variiert werden. Durch diese definierte und bewusste Inhomogenisierung der Anordnung der allein entlang der Längsausdehnung des Beobachtungsfensters angeordneten Lichtaustrittsflächen gelingt es die gewünschte Homogenisierung der Lichtverteilung zu erreichen. Insbesondere hat es sich bewährt, die Lichtleiterendflächen in den Lichtaustrittsöffnungen so anzuordnen und auszubilden, dass das Licht aus diesem in unterschiedliche Richtungen in den Arbeitsbereich bzw. Beleuchtungsbereich und hier in einen vorgegebenen Raumwinkelbereich fällt, so dass dieser Bereich möglichst homogen ausgeleuchtet wird. Durch diese Differenzierungen der Ausleuchtrichtungen, die eine Inhomogenisierung der Ausleuchtrichtungen beinhaltet, wird überraschender Weise die gewünschte verbesserte Homogenisierung der Ausleuchtung erreicht.

Durch die erfindungsgemäße Anordnung der Lichtaustrittsöffnungen ausschließlich entlang der Längsausdehnung des Beobachtungsfensters gelingt es die Anzahl der Austrittsöffnungen relativ hoch zu wählen und dadurch eine sehr gute Helligkeit zu erreichen bei erfindungsgemäß guter Homogenität. Das erfindungsgemäße Endoskoprohr erweist sich damit als sehr geeignet für schwierige Operationen, die eine sehr verlässliche und gute Beobachtung des Arbeitsraumes benötigen. Als besonders vorteilhaft hat es sich bewiesen, die vorgenannten beiden Konzepte, einmal die inhomogene Verteilung der Lichtaustrittsöffnungen entlang der Längsausdehnung des Beobachtungsfensters und zum anderen die inhomogene Wahl der Abstrahlrichtungen aus den Lichtleiterendflächen der Lichtaustrittsöffnungen zu kombinieren. Durch diese Kombination wird ein besonders effizientes und homogenes Bestrahlen des relevanten Arbeitsbereiches ermöglicht. Durch diese Kombination lassen sich die Vorteile beider Konzepte verbinden und zudem eine deutlich engere Anordnung der Lichtaustrittsöffnungen und damit entweder eine Verkleinerung des Durchmessers des Endoskoprohres bei vorgegebener abgegebener Lichtmenge zur Beleuchtung des relevanten Arbeitsbereiches ermöglichen oder umgekehrt bei vorgegebenen Durchmesser des Endoskoprohres eine gesteigerte abgegebene Lichtmenge erreichen. Beides führt zu einem erweiterten möglichen Einsatzbereich des erfindungsgemäßen Endoskoprohres gegenüber dem bisher bekannten Endoskoprohres.

Dabei hat es sich besonders bewährt, die Festlegung der Abstrahlrichtung des Lichtes aus den Lichtaustrittsöffnungen dadurch festzulegen, dass ein Prisma im Bereich der Lichtleiterendflächen bzw. ein holografisches Element angeordnet ist, durch die die gewünschte Änderung der Abstrahlrichtung relativ zur Lichtaustrittsöffnung aus den Lichtleiterendflächen festgelegt ist. Durch diese Anordnung eines Prismas oder eines holografischen Elementes gelingt es sehr definiert und in vorgegebener Weise durch die Wahl des Prismas bzw. die Ausbildung des holografischen Elementes eine verlässliche und sichere Umlenkung des aus dem Lichtleiter ausgetretenen Lichtstrahls in die gewünschte Richtung zur Schaffung einer möglichst homogenen Teilung zu gewährleisten. Dabei hat es sich besonders bewährt, ein Prisma oder ein holografisches Element für mehrere benachbarte Lichtleiterendflächen gemeinsam zu verwenden. Hierdurch ist einerseits eine sehr kompakte Bauweise und einfache Herstellung und sichere und gleichmäßige Ablenkung erreicht. Dies führt zu einer sehr kostengünstigen Lösung des erfindungsgemäßen Endoskoprohrs. Alternativ bzw. zusätzlich hat es sich bewährt, die Endflächen des bzw. der Lichtleiter so auszubilden, dass die Fläche die Längsachse des Lichtleiters unter einem Winkel ungleich 90° schneidet. Durch die Wahl des Winkels lässt sich eine zielgerichtet Ablenkung des austretenden Lichts in die gewünschte Richtung und differenziert nach Lichtaustrittsöffnung erreichen. Als besonders vorteilhaft hat es sich dabei erwiesen, mehrere Lichtleiter zu einem Lichtleiterbündel zusammen zu fassen und diese gemeinsam in der Lichtausgangs-öffnung miteinander zu verkleben und die Endfläche dieses Verbundes aus Lichtleiterenden und Kleber gemeinsam so insbesondere durch Schleifen so auszubilden, dass die Oberfläche flächig teilweise abgetragen und der gewünschte Winkel der Lichtleiterendflächen gegenüber der Längsausdehnung der Lichtleiter erreicht wird. Durch diese Ausbildung ist einerseits sichergestellt, dass die Endflächen sehr effizient den Übergang vom optisch dichteren Material in die Umgebung in die gewünschte Abstrahlrichtung ermöglichen und andererseits ein sehr dichter Verschluss der Lichtaustrittsöffnung und damit des Innenraums des Endoskoprohrs ermöglichen. Durch diese erfindungsgemäße Dichtwirkung ist eine Sterilisierung in der Art einer Autoklavierung erst sinnvoll ermöglicht.

Als besonders vorteilhaft hat es sich erwiesen, zwischen den Lichtleiterendflächen und den Lichtaustrittsöffnungen das oder die Prismen bzw. das oder die holografischen Elemente anzuordnen und diese gemeinsam mit den Lichtleitern einheitlich und gemeinsam zu verkleben. Hierbei hat es sich bewährt, einen optisch inaktiven Kleber zu verwenden, der einen entsprechenden Brechungsindex hat wie die Lichtleiter bzw. das Prisma bzw. das holografische Element.

Durch die Ausbildung des Beobachtungsfensters in Form eines gewölbten, insbesondere zylindrisch gewölbten, Beobachtungsfensters das sich in Richtung der Längsausdehnung des Schaftes wölbt gelingt es, die Länge des Beobachtungsfensters und damit den Bereich zur Anordnung der Lichtaustrittsöffnungen zu erweitern und dadurch eine umfassendere homogene Beleuchtung des Arbeitsbereiches im Bereich vor und seitlich des distalen Endes des Endoskops sicher zu stellen. Durch die gewölbte Ausbildung des Endoskopfensters lässt sich auch der Beobachtungsbereich des Endoskops durch die entsprechende Ausbildung der Optik unterhalb des Beobachtungsfensters deutlich erweitern. Es entsteht somit ein sehr einsatzfähiges Endoskoprohr.

Vorzugsweise werden in den Lichtaustrittsflächen Fassungshülsen vorgesehen, mittels derer die Ausrichtung der Lichtleiter und Lichtleiterendflächen erfolgt, so dass eine Justierung der gewünschten Abstrahlrichtung des Lichtes aus den Lichtaustrittsöffnungen auf einfache und verlässliche Weise erfolgen kann.

Im Folgenden wird die vorliegende Erfindung anhand von Zeichnungen eines ausgewählten Beispiels der Erfindung näher erläutert. Die Erfindung ist nicht auf dieses dargestellte Beispiel beschränkt.
Fig. 1 zeigt eine Seitenansicht des distalen Endes des erfindungsgemäßen Endoskoprohres.
Fig. 2 zeigt eine Frontalsicht des distalen Endes des erfindungsgemäßen Endoskoprohres.
Fig. 3 zeigt schematisch die Ausbildung einzelner Lichtleiter mit unterschiedlich ausgebildeten Lichtleiterendflächen.

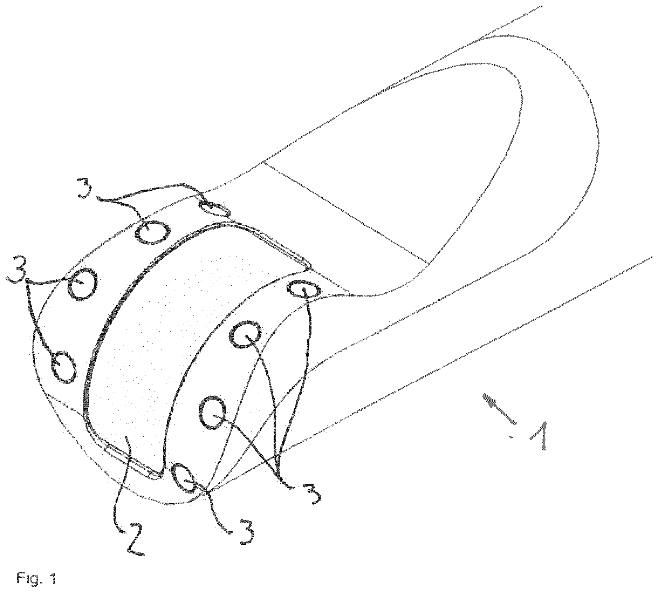

Fig. 1 zeigt in einer Schrägansicht das distale Ende 1 des erfindungsgemäßen Endoskoprohres. Zentral ist das Beobachtungsfenster 2, das langgestreckt ausgebildet ist und zentral im Bereich des distalen Endes 1 angeordnet ist. Das Beobachtungsfenster 2 ist dabei gewölbt dahingehend ausgebildet, dass die Wölbung ähnlich einem Zylinder in Längsrichtung des Endoskoprohres sich erstreckt. Das Beobachtungsfenster zeigt dabei eine im Wesentlichen rechteckige Gestalt, welche im Wesentlichen zylindrisch gewölbt ausgeführt ist.

Entlang der Längsausdehnung des Beobachtungsfensters 2 sind die Lichtaustrittsöffnungen 3 angeordnet. Es handelt sich hier um insgesamt 8 Lichtaustrittsöffnungen, die gegeneinander versetzt und unsymmetrisch zur Längsausdehnung zur Längsachse des Beobachtungsfensters angeordnet sind. Sie zeigen dabei unterschiedliche Abstände von der Längsachse bzw. sind abwechselnd versetzt entlang der Längsachse des Beobachtungsfensters 2 angeordnet. Die 8 Lichtaustrittsflächen sind dabei gleichartig ausgebildet. In diesen Lichtaustrittsflächen sind ein Bündel von Lichtleiterendflächen angeordnet, die miteinander verklebt ausgebildet sind, damit die Lichtaustrittsflächen Gas- und Flüssigkeitsdicht und damit autoklavierbar ausgebildet sind.

Durch die unsymmetrische Anordnung der Lichtaustrittsöffnungen 3 entlang des Beobachtungsfensters 2 gelingt es, einerseits das Licht aus den Lichtleiterendflächen in verschiedene Raumrichtungen insbesondere aufgrund der genannten Wölbung des Endbereiches mit dem Beobachtungsfenster 2 des distalen Endes 1 des erfindungsgemäßen Endoskoprohres abzustrahlen. Durch die versetzte Ausbildung gelingt es durch die inhomogene Anordnung der Lichtaustrittsöffnungen 3 eine homogenisiertere Ausleuchtung des Arbeits- und Beobachtungsraumes für einen mikroinvasiven Eingriff zu schaffen.

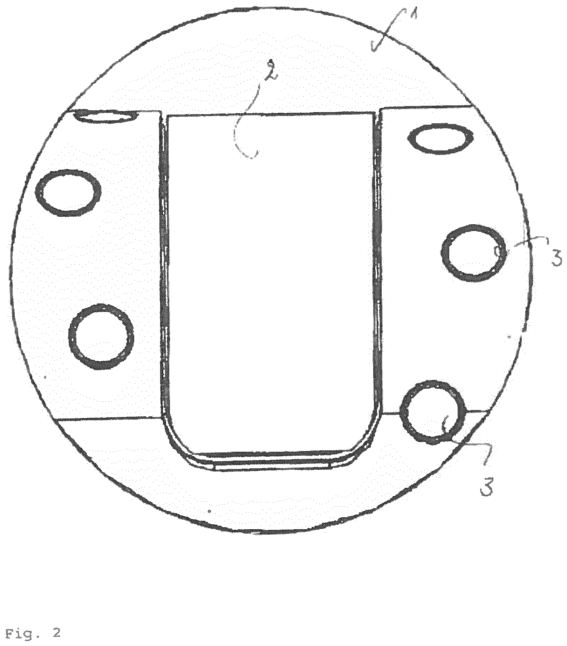

In Fig. 2 ist eine Ansicht von vorne auf das kreisrunde Endoskoprohr mit dem distalen Ende 1 und dem Beobachtungsfenster 2 beschrieben dargestellt. Seitlich mit unterschiedlichen Abständen sind entlang der Längsausdehnung des Beobachtungsfensters 2 verschiedene Lichtaustrittsöffnungen 3 angeordnet. Durch die inhomogene Anordnung des Beobachtungsfensters 2 gelingt es die gewünschte Homogenisierung der Beleuchtung des Arbeitsraumes zu erreichen.

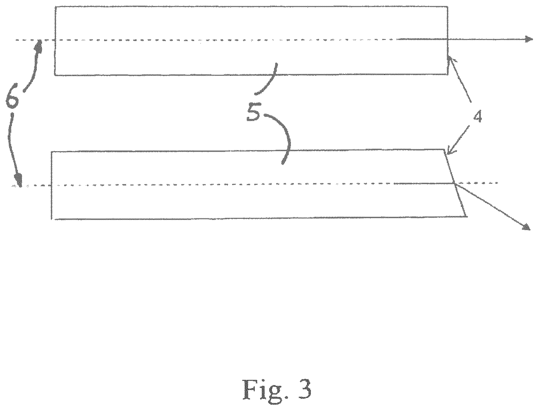

In Fig. 3 sind zwei beispielhafte Lichtleiter 5 dargestellt. Die Lichtleiter 5 enden in Lichtleiterendflächen 4. Die Lichtleiterendflächen 4 stellen plane Flächen dar, die in einem definierten Winkel zu der Längsausdehnung des Lichtleiters 5 stehen. Als besonders bevorzugt hat es sich erwiesen, einzelne oder alle Lichtleiterendflächen 4 in einem Winkel ungleich 90° auszubilden und dadurch eine Ausstrahlung des ausgesandten Lichtes schräg bzw. in einem vorgegebenen Winkel ab von der Längsausdehnung des Lichtleiters zu gewährleisten. Durch die Wahl des Winkels lassen sich sehr differenzierte Ausleuchtszenarien zur Erreichung der möglichst homogenen Beleuchtung des Arbeitsbereiches schaffen.

## Patentansprüche

1. Endoskoprohr mit einem zentralen, langgestreckten Beobachtungsfenster am distalen Ende und mehreren neben dem Beobachtungsfenster angeordneten Lichtaustrittsöffnungen für Lichtleiterendflächen zur Ausleuchtung des durch das Beobachtungsfenster beobachteten Raumwinkelbereichs, wobei die Lichtleiterendflächen in den Lichtaustrittsöffnungen so gehalten sind, dass Licht aus den Lichtleiterendflächen in unterschiedliche Richtungen des Raumwinkelbereichs strahlt, wobei das distale Ende des Schafts in Richtung der Längsausdehnung des Beobachtungsfensters, gewölbt insbesondere zylindrisch gewölbt ist, und
mehrere Lichtleiter zu einem Lichtleiterbündel zusammengefasst und diese gemeinsam in der Lichtaustrittsöffnung miteinander verklebt sind, und die Endfläche dieses Verbundes aus Lichtleiterenden und Kleber gemeinsam so ausgebildet ist, dass die Oberfläche flächig teilweise abgetragen ist und die Endflächen der Lichtleiter die Längsachse der Lichtleiter unter einem Winkel ungleich 90° schneiden.

2. Endoskoprohr nach Anspruch 1, **dadurch gekennzeichnet, dass** im Bereich der Lichtleiterendflächen wenigstens ein Prisma vorgesehen ist.

3. Endoskoprohr nach Anspruch 2, **dadurch gekennzeichnet, dass** das Prisma zwischen den Lichtleiterendflächen und den Lichtaustrittsöffnungen angeordnet ist.

4. Endoskoprohr nach einem der vorhergehenden Ansprüche, **dadurch gekennzeichnet, dass** Licht an den Lichtleiterendflächen gebrochen wird.

5. Endoskoprohr nach einem der vorhergehenden Ansprüche, **dadurch gekennzeichnet, dass** ein holographisches Element im Bereich der Lichtleiterendflächen zur Erzeugung von Strahlen in unterschiedlichen Richtungen vorgesehen ist.

6. Endoskoprohr nach Anspruch 5 **dadurch gekennzeichnet, dass** das holographische Element zwischen den Lichtleiterendflächen und den Lichtaustrittsöffnungen angeordnet ist.

7. Endoskoprohr nach einem der vorhergehenden Ansprüche, **dadurch gekennzeichnet, dass** in die Lichtaustrittsöffnungen Fassungshülsen zur Ausrichtung der Lichtleiterendflächen
eingesetzt sind.

## Claims

1. Endoscope tube comprising a central, elongate observation window at the distal end and a plurality of light-emergence openings, disposed next to the observation window, for optical waveguide end faces for illuminating the solid angle range observed through the observation window, wherein the optical waveguide end faces are held in the light-emergence openings in such a way that light radiates from the optical waveguide end faces into different directions of the solid angle range, wherein the distal end of the shaft is arched, more particularly cylindrically arched, in the direction of longitudinal extent of the observation window and a plurality of optical waveguides are combined to form an optical waveguide bundle and, together, these are adhesively bonded to one another in the light-emergence opening, and the end face of this grouping of optical waveguide ends and adhesive together is embodied in such a way that the surface is partly removed over an area and the end faces of the optical waveguides intersect the longitudinal axis of the optical waveguides at an angle not equal to 90°.

2. Endoscope tube according to Claim 1, **characterized in that** at least one prism is provided in the region of the optical waveguide end faces.

3. Endoscope tube according to Claim 2, **characterized in that** the prism is disposed between the optical waveguide end faces and the light-emergence openings.

4. Endoscope tube according to any one of the preceding claims, **characterized in that** light is refracted at the optical waveguide end faces.

5. Endoscope tube according to any one of the preceding claims, **characterized in that** a holographic element is provided in the region of the optical waveguide end faces for producing beams in different directions.

6. Endoscope tube according to Claim 5, **characterized in that** the holographic element is disposed between the optical waveguide end faces and the light-emergence openings.

7. Endoscope tube according to any one of the preceding claims, **characterized in that** socket sleeves are inserted into the light-emergence openings for aligning the optical waveguide end faces.

## Revendications

1. Tube d'endoscope comportant une fenêtre d'observation centrale allongée à l'extrémité distale et plusieurs ouvertures de sortie de lumière, disposées à proximité de la fenêtre d'observation, pour des surfaces d'extrémité de guides de lumière afin d'éclairer la plage d'angles solides observée à travers la fenêtre d'observation, dans lequel les surfaces d'extrémité des guides de lumière sont maintenues dans les ouvertures de sortie de lumière de manière à ce que la lumière provenant des surfaces d'extrémité des guides de lumière soit émise dans différentes directions de la plage d'angles solides, dans lequel l'extrémité distale de l'arbre est incurvée, en particulier de façon cylindrique, dans la direction de l'extension longitudinale de la fenêtre d'observation, et
plusieurs guides de lumière sont combinés en un faisceau de guides de lumière et ceux-ci sont collés ensemble dans l'ouverture de sortie de lumière, et la surface d'extrémité de ce composite est réalisée à partir d'extrémités de guides de lumière et d'adhésif de telle manière que la surface soit partiellement enlevée de manière plane, et
les surfaces d'extrémité des guides de lumière coupent l'axe longitudinal des guides de lumière selon un angle différent de 90°.

2. Tube d'endoscope selon la revendication 1, **caractérisé en ce qu'**il est prévu au moins un prisme dans la zone des surfaces d'extrémité des guides de lumière.

3. Tube d'endoscope selon la revendication 2, **caractérisé en ce que** le prisme est disposé entre les surfaces d'extrémité des guides de lumière et les ouvertures de sortie de lumière.

4. Tube d'endoscope selon l'une des revendications précédentes, **caractérisé en ce que** la lumière est réfractée au niveau des surfaces d'extrémité des guides de lumière.

5. Tube d'endoscope selon l'une des revendications précédentes, **caractérisé en ce qu'**il est prévu dans la zone des surfaces d'extrémité des guides de lumière un élément holographique destiné à générer des faisceaux dans différentes directions.

6. Tube d'endoscope selon la revendication 5, **caractérisé en ce que** l'élément holographique est disposé entre les surfaces d'extrémité des guides de lumière et les ouvertures de sortie de lumière.

7. Tube d'endoscope selon l'une des revendications précédentes, **caractérisé en ce que** des manchons de fixation destinés à l'orientation des surfaces d'extrémité des guides de lumière sont insérés dans les ouvertures de sortie de lumière.
